impossible to produce cellulose I, unless bundles of non-crystalline glucan chains were already oriented in parallel fashion. Thus, it is logical that the orientation takes place because of the undirectional polymerization of multiple glucan chains. We know that a basic unit of approximately 17 - 20 Angstrom is produced by the isolated enzyme system (see Fig. 3). This fibril is in itself too small to have any appreciable crystallinity, and thus, the electron diffraction pattern of in vitro cellulose shows an amorphous pattern (Fig. 4).

A strain of A. xylinum used for the in vitro studies was supplied by M. Benziman, Hebrew University, Jerusalem, Israel, and it was the same strain used by Benziman's group for their studies on cellulose synthase. Guanyl oligonucleotide was prepared as described (Ross, et al., (1985) FEBS Lett 186 191). Incubations, containing 75 mM tris (pH 8.6), 15 mM MgCl2, 10 mM UDP-glucose, I mM CaCl2, I mM EDTA, 0.5 percent digitonin, and about 20 ug/ml of guanyl oligonucleotide, were caried out in Eppendorf pipette tips at room temperature for 20 to 30 minutes. A small drop of the reaction product was then placed on a carbon-coated Formvar copper grid, drained, and stained with I percent aqueous uranyl acetate containing 0.1 mg/ml bacitracin. We have succeeded in preparing a solubilized enzyme preparation and activator from two other strains of Acetobacter that produced fibrils of similar size and morphology.

When the in vitro synthesized material is treated with an aqueous solution comprising a suitable agent to remove non-cellulosic components, a precipitate will form which may be washed with distilled water.

Aqueous solutions comprising suitable agents are capable of dissolving non-cellulosic components or contaminants and are often characteristically solvents for proteinaceous materials. The treatment of the cellulose synthesized in vitro to remove included contaminants results in a physical rearrangement of the cellulose intermolecular structure to form cellulose I or crystalline polymorphs thereof. Agents dissolved in aqueous solutions and usable in the practice of the present invention include detergents, acidic substances and alkaline substances. Alkaline aqueous solutions preferably comprising a hydroxide of an alkali metal in a concentration between about 2 weight percent and about 6 weight percent are one type of preferred alkaline aqueous solution. Acidic substances are preferably mineral acids in a strength between about 0.5 N and about 1.0 N.

Aqueous solutions of detergent, for example ALCONOX or sodium dodecyl sulfate at concentrations between about 2 weight percent and about 10 weight percent, are also preferred aqueous solutions comprising an agent facilitating the solubilization of proteinaceous materials and precipitation of the cellulose. Treating the cellulose synthesized in vitro with an aqueous solution comprising an agent as described above at a temperature between about 70°C and about 120°C and for a period between about 10 minutes and about 90 minutes is preferred to precipitate the cellulose synthesized in vitro as cellulose I or a crystalline polymorph thereof. This cellulose I or crystalline polymorph thereof may next be washed, preferably with water or purified water, for use or further processing.

The treated and washed material exhibits an electron diffraction pattern typical of the native cellulose I polymorph (Fig. 6). To obtain the cellulose used in Figures 5-8, cellulose synthesized in vitro was treated with an aqueous solution having 4 weight percent sodium hydroxide. The treatment was at 100°C for 30 minutes and was followed by washing with distilled water. Similar electron diffraction patterns were obtained when sodium dodecyl sulfate at 5 weight percent or 10 weight percent was used in place of the sodium hydroxide and the treatment and washing were repeated once.

For electron diffraction studies, a $MoO_3$ standard was used to calibrate the d spacings. For controls, cellulose produced by A. xylinum was cleaned with 1N HCl and then a mixture of methanol and chloroform (1:1 by volume), washed with DD $H_2O$, frozen in liquid nitrogen, ground to a fine powder, and dried onto carbon-coated grids. The in vitro material was placed directly onto grids and air-dried. For electron diffraction analysis, a transmission electron microscope (Phillips EM 420) was operated at 120 kV with a condenser aperture of 30 um, the condenser-1 lens current set at position "5" in microprobe position, and a beam current of less than 5 uA. For exposure, the low-dose technique of beam shift was employed; the diffraction pattern was recorded on electron microscope film (Mitsubishi MEM, from Japan) and developed (Mitsubishi Gekkol developer) for 4 minutes at 20°C.

The average crystallite size as determined by line broadening, was approximately 39 - 45 Angstroms for the 200 reflection, 38 - 44 Angstroms for the 110 reflection, and 39 - 45 Angstroms for the 110 reflection (see Fig. 6). These data are in line with the observed dimensions of the flattened fibrillar form of approximately 36 - 54 Angstroms (see Fig. 5). Thus, two or three of the 17 Angstrom fibrils must come together upon alkali treatment to produce a crystalline microfibril which exhibits the same polymorph as native cellulose I. It would not have been expected by one ordinarily skilled in the art that treatment of the in vitro-produced cellulose with sodium hydroxide, or an agent to remove non-cellulosic materials, would result in the production of native cellulose I.

Samples of cellulose synthesized in vitro and washed with 4 weight percent NaOH, untreated samples of cellulose synthesized in vitro and cellulose synthesized in vivo by Acetobacter xylinum were subjected to electron diffraction as described above. Table I shows the transverse dimensions of crystallites in in vitro and in vivo cellulose.

_Acetobacter xylinum_ is a preferred source of the cell-free system. The cellulose I produced by the processes of the present invention is similar to cotton fiber in microscopic appearance.

Figure I shows an _in vivo_ synthesized cellulose ribbon from an intact cell of _Acetobacter xylinum._

Figure 2 shows an electron diffraction pattern of _in vivo_ synthesized cellulose of _Acetobacter xylinum._ Meridional reflections are vertical and equatorial reflections, horizontal.

Figure 3 shows an electron microscopic view of unprocessed _in vitro_ synthesized cellulose replete with I7 Angstrom microfibrillar particles (magnification of about 75,000 ×).

Figure 4 shows an electron diffraction pattern of unprocessed _in vitro_ synthesized cellulose. The line broadening pattern suggests an amorphous, noncrystalline, _in vitro_ product.

Figure 5 shows an electron micrograph of _in vitro_ synthesized cellulose processed by alkali treatment to remove non-cellulosic materials. This approximately I35,000 × magnification reveals an ordered bundle of cellulose microfibrils.

Figure 6 shows an electron diffraction pattern of alkali-treated _in vitro_ synthesized cellulose. Meridional reflections are vertical and equatorial reflections, horizontal. This pattern indicates that the cellulose is ordered and is crystalline as compared to the _in vitro_ synthesized cellulose in Figure 3 and Figure 4.

Figure 7 shows an electron micrograph of _in vitro_ synthesized cellulose as described in Figure 5 with aggregates having a diameter of about 2.3 microns. Aggregates as large as I0 microns have been observed.

Figure 8 shows an autoradiograph of cellulose synthesized _in vitro_ in the presence of $^3$H-UDP-glucose. The association of radioactivity with the _in vitro_-synthesized processed cellulose confirms its cellulosic nature.

The _in vitro_ synthesis of microbial cellulose may be advantageously accomplished by a process involving the cultivation of a cellulose-producing microorganism in a nutrient medium comprising an amount of a cellulase preparation sufficient to hydrolyze cellulose being produced by the cellulose-producing microorganism. Cellulose-producing cells such as _Acetobacter xylinum_ cells are inoculated in the cellulase preparation-containing nutrient medium and aerobically incubated by shake culture to facilitate the production of a dense cell culture. The cells are harvested and treated to produce a detergent-solubilized active enzymic component and a crude supernatant retaining biological activity. The crude supernatant and detergent-solubilized enzymic component are then combined in a solution with UDP-glucose and incubated to facilitate the _in vitro_ synthesis of cellulose.

Cellulose-producing microorganisms of the species _Acetobacter xylinum_ (for example, ATCC No. 23769 from the American Type Culture Collection, Rockville, MD) were used as a source of cellular components having cellulose-synthetic capacity. Microorganism samples were seeded on the surface of Schramm Hestrin agar and grown for 5-7 days. Cells from a single colony were transferred to flasks containing 200 ml Schramm Hestrin nutrient medium and 50 ul Celluclast (a cellulolytic preparation from Novo Industri A/S, Denmark). The inoculated medium was incubated at 28°C for 36 hr on a rotary shaker set at I20 rpm. Cells were collected by passage of the culture medium through I0 layers of cheesecloth and centrifugation at I0,000 × g for I0 minutes. The centrifuged cells were washed twice with cold TME buffer (50mM Tris-HCl (pH 7.5), I0mM MgCl$_2$, ImM EDTA) to remove Celluclast. Cells from each flask were resuspended in 5 ml TME and the optical density (OD) at 660 nm was determined (40 ug = 0.02 OD). After recentrifugation at I0,000 × g for I0 min the cell pellets were resuspended in TME containing 20% polyethylene glycol (TME-PEG). About I5 ml of TME-PEG for each mg of dry cell weight was used to prepare this cell suspension.

The cell suspension was passed slowly through a French pressure cell at I6,000 psi. The resultant homogenate was centrifuged at I2,000 × g for I0 min at 4°C. The pellet thus produced was resuspended in TME (I/4 the volume of TME-PEG used earlier). This resuspension was centrifuged at I8,000 × g for 20 min at 4°C to obtain a supernatant (SF) and a pellet. The pellet was resuspended in 50 mM Tris-HCl(pH7.5), 22 mM MgCl$_2$, I mM EDTA (S-TME) (having a volume of from about I/I6 to about 3/32 of the TME-PEG volume first used herein) to form a resuspended pellet (MF). A crude preparation was made by first preparing a mixture containing: I0 ml of SF; I.25 ml of I0 mM GTP; I25 ul of 0.IM CaCl$_2$ and I.25 ml TME. The mixture was incubated at 37°C for 2 hours, then heated to I00°C for 3 min. After centrifugation to remove coagulated substances, a supernatant was obtained (crude Gx), frozen in liquid nitrogen and stored at -20°C.

The MF (resuspended pellet) was used to prepare a digitonin-solubilized enzymic component. The MF was suspended in S-TME and mixed with the detergent, digitonin, to have a final protein concentration of 6.25 mg/ml and I% digitonin. This mixture was sonicated for 5 min at 4°C and then shaken gently for 30 min at this temperature. The resultant mixture was then centrifuged at I00,000 × g for I hr at 4°C. The supernatant resulting was a digitonin-solubilized preparation (DS) which comprised cellulose synthase.

Equal volumes of DS and crude Gx were mixed and UDP-glucose was added to a final concentration of 20 mM. This mixture was incubated at 30°C to produce the _in vitro_ synthesis of cellulose. For a more detailed description of these procedures see Lin et al. (I985), Science, V 230, pp 822-825.

In this invention, cellulose is generated by an isolated enzyme system, according to procedures described above. An important aspect of this invention is that the material produced may be treated with an agent which removes non-cellulosic materials, thus allowing the _in vitro_ synthesized cellulose to associate in such a manner as to produce crystalline cellulose I microfibrils. In order for this to take place, the _in vitro_ synthesized fibrils must have been vectorially synthesized and remain in relatively close position to one another to ensure intermolecular hydrogen bonding when the non-cellulosic substances are removed. It would be theoretically

Europäisches Patentamt

European Patent Office

Office européen des brevets

(11) Publication number: **0 260 093**
**A2**

(12) **EUROPEAN PATENT APPLICATION**

(21) Application number: **87307891.9**

(22) Date of filing: **07.09.87**

(51) Int. Cl.⁴: $C\ 12\ P\ 19/04$
//(C12P19/04,C12R1:02)

(30) Priority: **08.09.86 US 905481**

(43) Date of publication of application:
**16.03.88  Bulletin  88/11**

(84) Designated Contracting States:
**AT BE CH DE ES FR GB GR IT LI LU NL SE**

(71) Applicant: **BOARD OF REGENTS THE UNIVERSITY OF TEXAS SYSTEM**
**201 West 7th Street**
**Austin Texas 78701  (US)**

(72) Inventor: **Brown, R. Malcolm**
**305 Skyline Drive**
**Austin Texas 78746  (US)**

**Lin, Fong-Chyr**
**3555 A Lake Austin Blvd.**
**Austin Texas 78703  (US)**

(74) Representative: **Perry, Robert Edward et al**
**GILL JENNINGS & EVERY 53-64 Chancery Lane**
**London WC2A 1HN  (GB)**

(54) **Methods for production of cellulose I involving in vitro synthesis.**

(57) Cellulose synthesized in vitro by a cell-free system, preferably from a cellulose-producing microorganism, is freed from contaminating components such as proteins, metabolites or lipids, to form cellulose I or a crystalline polymorph. The cellulose I is similar to cotton fiber in microscopic appearance.

Fig. I

## Description

## METHODS FOR PRODUCTION OF CELLULOSE I INVOLVING IN VITRO SYNTHESIS

The present invention relates to the in vitro production of cellulose and treatment of cellulose synthesized in vitro to produce cellulose I or crystalline polymorphs thereof.

In vitro synthesis of cellulose by cellular extracts from cellulose-producing microorganisms has been accomplished and investigated by a few laboratories (see, for example, Ross et al. (1986) Carboh. Res. V 149 pp 101-117 and Lin et al. (1985) Science V 230 pp 822-825). The physical nature of cellulose so produced has yet to be completely defined. Before the discoveries described herein and comprised by the present invention, the in vitro cellulose product was obtainable only in the form of fibrils which were observed to be shorter and narrower than those produced by normal in vivo microbial cellulose biosynthesis.

Cellulose, a polymer of 1,4-beta-D-glucan, is the most abundant biopolymer on earth. It is the primary component of such products as wood, cotton, and paper. Cellulose exists in nature as microfibrils, which form the structural framework of the cell walls of plants, some algae, and certain fungi. Among the few bacteria that synthesize cellulose, Acetobacter xylinum has been studied intensely because it produces abundant amounts in growth medium. The cellulose is extruded from a row of pores aligned along the longitudinal axis of the cell and forms an extracellular pellicle, which consists of ribbons of cellulose microfibrils. Freeze etching of A. xylinum cells has revealed in the outer member a row of particles, complementary to the pores, which may take part in the synthesis of cellulose.

Many attempts have been made to synthesize cellulose in vitro, but only recently, by means of membrane fractions from A. xylinum (Aloni et al. (1982) Proc. Nat'l. Acad. Sci V 79 p 6408), has a convincing high-rate synthesis of a 1,4-beta-D-glucan polymer been achieved. These studies have demonstrated a complex regulation of cellulose synthase, mediated by GTP (guanosine 5'-triphosphate) or related substances, in which this enzyme is solubilized in a form that still possesses its regulatory properties (Aloni et al (1983) J. Biol. Chem. V 258, p 4419). Further studies have clarified the role of GTP. It is converted to an unusual guanyl oligonucleotide by an activator-forming enzyme distinct from cellulose synthase, and this derivative, tentatively characterized as a cyclic oligonucleotide composed of GMP (guanosine 5'-monophosphate) residues, is the true activator of cellulose synthase. The in vitro product is clearly an alkali-insoluble 1,4-beta-D-glucan polymer (Aloni et al. (1982) Proc. Nat'l. Acad. Sci V 79, p 6408 Aloni et al. (1983) J. Biol. Chem. V 258, p 4419 and Ross et al. (1986) Carbohyd. Res. V 149, pp 101-117); however, no information has become available on the crystalline structure or microfibrillar nature of the product.

Native celluloses exist in a polymorphic form known as cellulose I or crystalline polymorphs thereof (R.D. Preston. The Physical Biology of Plant Cell Walls, Chapman & Hall, London, 1974). This polymorph is known to contain glucan chains that are parallel to each other. The cellulose I polymorph is characteristic of all native celluloses synthesized by living systems to date. Cellulose I is a metastable polymorph; it exists in a more thermodynamically unstable form than that of the so-called cellulose II polymorph. In this form, the glucan chains are antiparallel to one another, and there is one additional hydrogen bond linking each glucose residue. This form of cellulose is thermodynamically more stable. Cellulose II is formed only when cellulose I is dissolved and reprecipitated. Cellulose II does not exist in nature. This is because the terminal synthesizing enzymes (Brown et al, J. Appl. Polymer Sci., Appl. Polymer Symposium, 37: 33-78. 1983) involved in cellulose synthesis appear to modulate a vectored or unidirectional pattern of polymerization. The result is that the cellulose is synthesized naturally in the form of parallel glucan chains. The catalytic sites on the enzyme complex are close enough that the nascent glucan chains have a great probability of forming intermolecular hydrogen bonds, to form the basic crystalline structural unit of native cellulose known as the microfibril.

Native celluloses also are synthesized in a crystalline form. This can be proved by electron diffraction analysis. The electron diffraction pattern is characteristic for the type of cellulose polymorph form. That is, cellulose I is a polymorph that has characteristic electron diffraction patterns different from those of cellulose II. At the ultrastructural level, the crystalline form of cellulose can be visualized by a variety of electron microscope techniques, among them negative staining. This technique is useful in that it can identify small aggregates of glucan chains down to a mean diameter of approximately 12 - 15 Angstroms.

It is important to note that prior to this invention, no polymorphs of crystalline cellulose I are known to have been synthesized in vitro. By this, it is meant that the actual enzyme systems for cellulose synthesis have been isolated and removed from the intact cell and induced to form the cellulose by the appropriate addition of substrate and the use of appropriate incubation termperatures and conditions. Thus, this invention is the first report of the in vitro synthesis of a crystalline cellulose I polymorph. This means that this form of cellulose is identical to that formed by in vivo systems; however, with this invention, there are available, additional parameters that would enable one to control, at will, the crystallization phenomenon.

The present invention comprises a process for converting cellulose synthesized in vitro to cellulose I or a crystalline polymorph thereof. The process consists essentially of treating the cellulose with an aqueous solution comprising an agent which facilitates the solubilization of proteinaceous materials or lipids and precipitation of the cellulose as cellulose I or a crystalline polymorph thereof. The agent is preferably an alkaline substance, an acidic substance or a detergent. Once cellulose synthesized in vitro by a cell-free system, preferably from a cellulose-producing microorganism, has been freed from contaminating components such as proteins, metabolites, or lipids, it forms cellulose I or a crystalline polymorph.

TABLE 1

Transverse dimensions of crystallites in in vitro
and in vivo cellulose from Acetobacter xylinum

| Source | Equatorial electron reflection investigated (Angstrom units) | | |
|---|---|---|---|
| | 6.0 | 5.3 | 3.9 |
| in vitro cellulose before alkali treatment | absent | d=4.7-5.2 15-19 | absent |
| in vitro cellulose after alkali treatment | 43 | 44 | 38 |
| in vivo (native) cellulose | 63-85 | 54-72 | 50-52 |

Typically, native crystalline celluloses have been cleaned with sodium hydroxide, but generally at concentrations of 24 weight percent. With this concentration, the glucan chains are solubilized by the sodium hydroxide, and when the sodium hydroxide is removed, the cellulose is reprecipitated, this time to form antiparallel glucan chains, resulting in another cellulose polymorph known as cellulose II). This so-called mercerized cellulose is largely used in industry and represents the more thermodynamically stable form of cellulose. This would be the most obvious way to treat cellulose, to obtain a reprecipitated form. Those skilled in the art have not believed that cleaning in vitro cellulose with low concentrations of sodium hydroxide or other solvents such as those containing acids or detergents which would remove components such as non-cellulosic carbohydrates, other proteins or enzymes, the cellulose synthases themselves, digitonin, or other components in the reaction mixture, would cause formation of cellulose I or its crystalline polymorphs.

It was also found that other specific treatments were capable of causing conversion of cellulose synthesized in vitro to cellulose I or its crystalline polymorphs. For example, when cellulose synthesized in vitro as described above was treated with the detergent ALCONOX (Alconox, Inc., N.Y., N.Y. I0003) such conversion was noted by observation of product cellulose with an electron microscope. The cellulose synthesized in vitro was incubated with an aqueous solution containing 2 weight percent ALCONOX for 30 minutes at I00°C to produce the cellulose I or its crystalline polymorphs. It was additionally noted that acidic aqueous solutions could be used to facilitate the production of cellulose I or crystalline polymorphs thereof from cellulose synthesized in vitro. In a series of experiments, cellulose synthesized in vitro was subjected to 0.25 N, 0.50 N, I.0 N or 2.0 N aqueous hydrochloric acid for 30 minutes at I00°C. Under examination by electron microscopy as described above the ALCONOX-treated cellulose showed fibers appearing larger than those of cellulose synthesized in vitro, and slightly smaller than cellulose washed with an aqueous alkaline solution and about the same size as cellulose washed with sodium dodecyl sulfate. The cellulose preparations washed with 0.5 N HCl and I.0 N HCl had fibrils significantly larger than untreated cellulose synthesized in vitro. The cellulose samples washed with 0.25 N HCl or 2.0 N HCl showed fibrils which appeared no larger than those of cellulose synthesized in vitro.

Thus, true cellulose I or crystalline polymorphs thereof, in the form of microfibrils, can be synthesized in vitro. If the procedure for enzyme preparation of Lin, et al. as described herein is used, the final crystallization step can be achieved only by treatment with an agent facilitating the solubilization of proteinaceous or other non-cellulosic materials and precipitating the cellulose. When methods are found to purify and immobilize cellulose-synthesizing enzymes, it should be possible that native cellulose I could be made directly in vitro. While this has not yet been proven possible, the embodiments of the present invention are meant to extend to the concept that crystalline cellulose I can be synthesized in vitro, provided no contaminating components interfere with the crystallization at the time polymerization takes place.

An important aspect of the present invention relates to the discovery that cellulose synthesized in vitro may be converted from a physically diffuse state into a more crystalline state by removal of contaminating non-cellulosic materials. This more crystalline state is exemplified by a fibrous structure characteristic of cellulose I or a crystalline polymorph thereof and similar to that of cotton fiber and should thus prove usable to manufacture materials having sufficient structural integrity for uses analogous to those of cotton, for example.

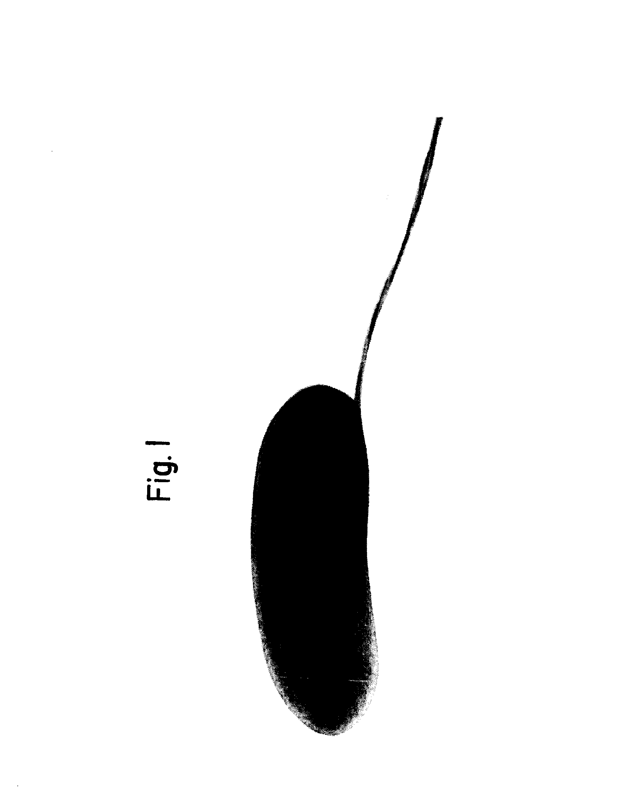

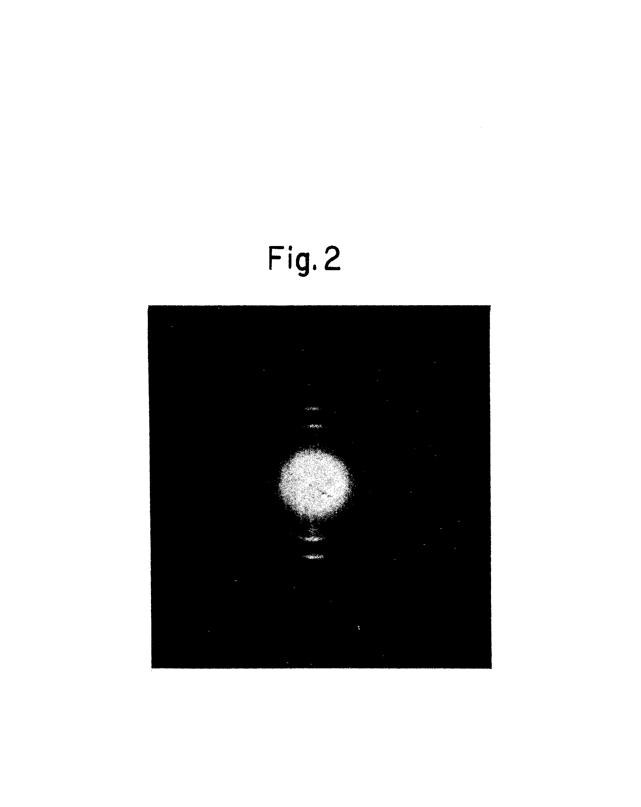

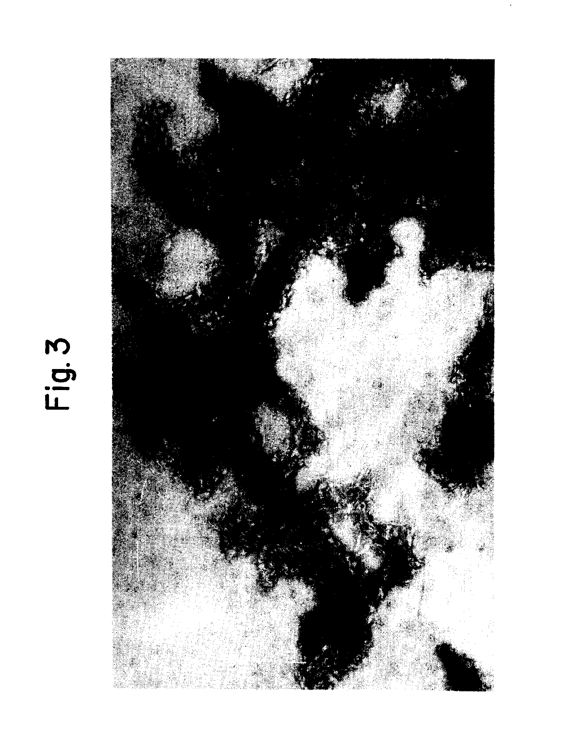

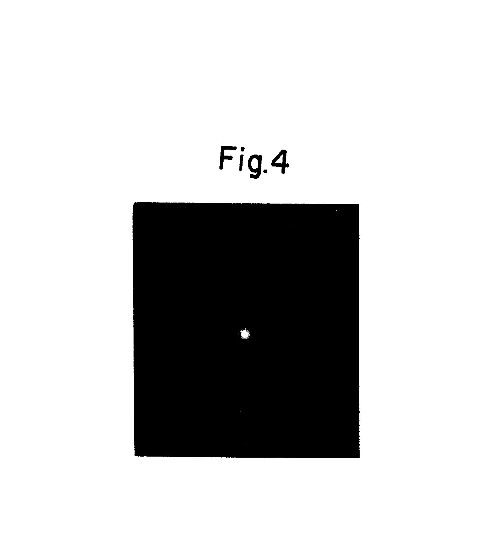

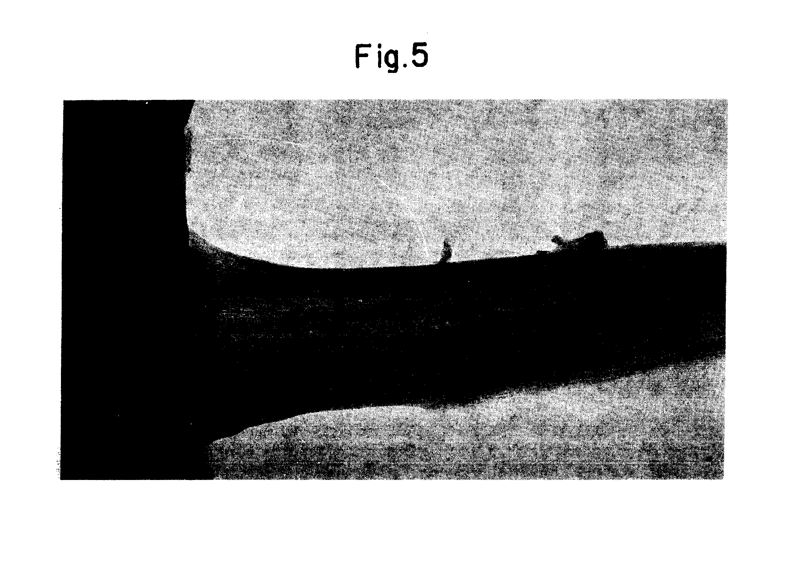

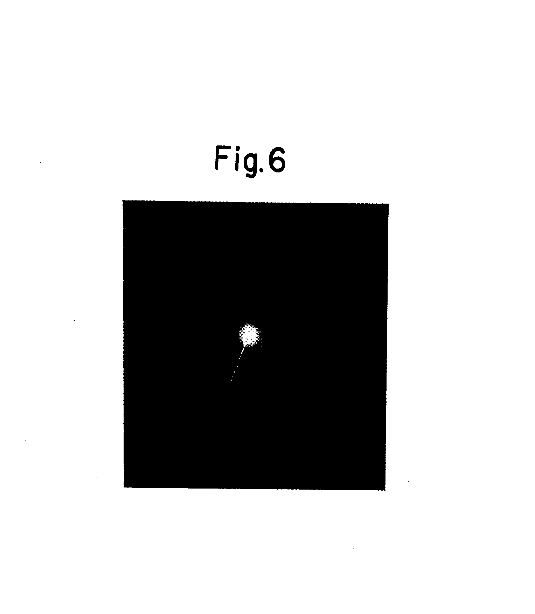

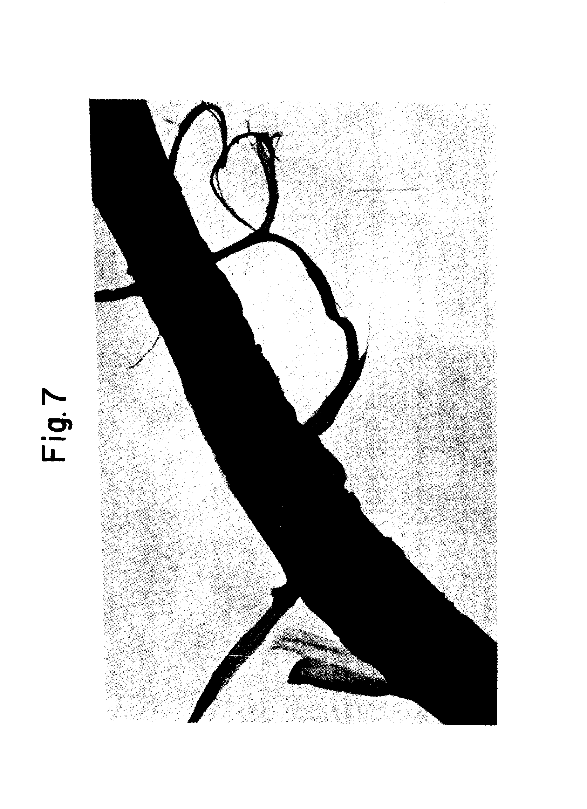

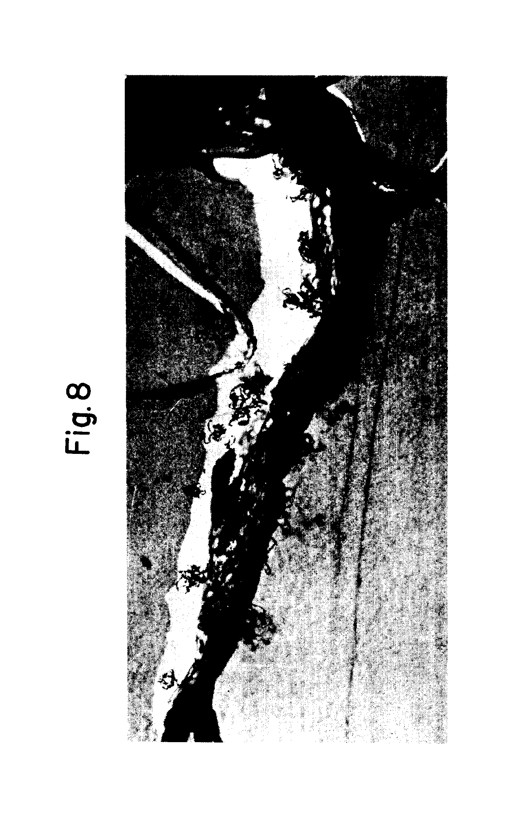

The incorporation of $^3$H-UDP-glucose into the product cellulose I is shown in Figure 8. For autoradiography, the grid was prepared as described (Chanzy, et al., (1984) FEBS Lett I72 I93), but with radioactive substrate. It was shadow-cast unidirectionally with platinum-carbon at a I0° angle. Then the grid was autoradiographed as reported (Kopriwa, Histochemie 37, I (I973). Although individual fibrils were not distinguished after shadowing, this procedure was found necessary to preserve the integrity of the in vitro product during the autoradiographic developing and fixing procedures.

Although the present invention primarily concerns in vitro cellulose synthesis by components of microorganism, the principles established herein should be applicable to in vitro cellulose synthesis by plant components when such plant component synthesize cellulose without the form of cellulose I or crystalline polymorphs thereof.

## Claims

1. A process for the production of cellulose I or a crystalline polymorph thereof, which comprises treating cellulose synthesized in vitro, to remove non-cellulosic materials.

2. A process according to claim 1, wherein the cellulose is synthesized by plant components.

3. The process of claim 1, wherein the cellulose is synthesized by components of a cellulose-producing microorganism.

4. A process according to claim 1, which comprises the steps of:

obtaining an enzyme preparation from a cellulose-producing microorganism, the preparation comprising cellulose synthase and any other enzyme necessary for cellulose synthesis;

incubating the enzyme preparation in an aqueous medium comprising an activator of cellulose synthesis and a metabolic precursor of cellulose; and

treating the thus-synthesized cellulose, to substantially remove contaminants thereform.

5. The process of claim 4, wherein the activator of cellulose synthesis comprises a guanyl nucleotide or a derivative thereof.

6. The process of any of claims 3 to 5, wherein the microorganism is Acetobacter xylinum.

7. The process of any preceding claim, which comprises treating the cellulose with an alkaline aqueous solution containing 2 to 6, e.g. about 4% by weight of an alkali metal, e.g. sodium hydroxide at 70 to 120°C 10 to 90, e.g. about 30 minuts, to precipitate the product.

8. The process of any of claims 1 to 6, which comprises treating the cellulose with an aqueous solution containing an agent facilitating the solubilisation of proteinaceous materials, and precipitating the product.

9. The process of claim 8, wherein the agent is a detergent, preferably sodium dodecylsulfate, e.g. in a concentration of 2 to 10% by weight.

10. The process of claim 8, wherein the agent is an acid, preferably a mineral acid such as hydrochloric acid, e.g. in a concentration of 0.5 to 1.0 N.

11. The process of any of claims 7 to 10, which comprises the additional step of washing the precipitated cellulose to substantially remove the aqueous alkaline solution or the agent, as appropriate, and preferably also to remove non-cellulosic proteinaceous materials and metabolites from the cellulose synthesized in vitro.

12. The process of claim 11, wherein the agent is a detergent and the treating and washing steps are repeated once in order.

0260093

Fig. 1

0260093

Fig. 2

Fig. 3

0260093

Fig.4

## Fig.5

0260093

Fig.6

## Fig. 7

## Fig. 8